# EUROPÄISCHE PATENTSCHRIFT

(11) **EP 2 820 965 B1**
(45) Veröffentlichungstag und Bekanntmachung des Hinweises auf die Patenterteilung: **13.02.2019**
(21) Anmeldenummer: 14176026.4
(22) Anmeldetag: 07.07.2014
(51) Int. Cl.: A43B 7/00, A43C 11/14, A61F 7/10, A61F 7/02, A41D 13/005

(54) **Fuß-Kühlmanschette zum Kühlen eines Fußes**
Foot cooling sleeve for cooling a foot
Manchette de refroidissement de pied destinée à refroidir un pied

(30) Priorität: 05.07.2013 DE 202013102980 U
(43) Veröffentlichungstag der Anmeldung: 07.01.2015
(73) Patentinhaber: Tutakhiel, Ahmad Jahn, 80939 München (DE)
(72) Erfinder: Tutakhiel, Ahmad Jahn, 80939 München (DE)
(74) Vertreter: Viering, Jentschura & Partner mbB Patent- und Rechtsanwälte

(56) Entgegenhaltungen:
- EP-A1- 2 380 534
- WO-A1-2005/074846
- GB-A- 2 261 822
- US-A1- 2002 038 098
- US-A1- 2007 100 264
- ROTHMAIER M ET AL: "Design and performance of personal cooling garments based on three-layer laminates", MEDICAL & BIOLOGICAL ENGINEERING & COMPUTING, SPRINGER, BERLIN, DE, Bd. 46, Nr. 8, 25. Juni 2008 (2008-06-25), Seiten 825-832, XP019864914, ISSN: 1741-0444, DOI: 10.1007/S11517-008-0363-6

## Beschreibung

### Gebiet

Die vorliegende Erfindung betrifft eine Fuß-Kühlmanschette zum Kühlen eines Fußes, und betrifft insbesondere eine Fuß-Kühlmanschette zum Kühlen eines Fußes, welche nach dem Verdunstungsprinzip (z.B. Wasserverdunstungsprinzip) arbeitet, d.h. eine Verdampfungswärme, welche dem Fuß zum Verdampfen/Verdunsten eines Kühlmittels (z.B. Wasser) entzogen wird, nutzt, um den Fuß zu kühlen. Mit anderen Worten stellt die Manschette dem Fuß eine zu verdunstende/verdampfende Menge an flüssigem Kühlmittel bereit, dessen Verdunstung zu einer Kühlung des Fußes führt/beiträgt. Bei dem Kühlmittel kann es sich insbesondere um Wasser, z.B. bevorzugt um Leitungswasser handeln. Der Fuß kann zusätzlich mittels eines in einer Tasche der Manschette bereitgestellten Kühlelements, wie zum Beispiel eines Kühlakkus, gekühlt werden.

### Hintergrund

Es ist bekannt, dass bei Verletzungen des Beines oder des Fußknöchels oder beim Vorliegen einer erhöhten Körpertemperatur eine Verringerung der Körpertemperatur im Bereich des Beines den Heilprozess bzw. das Wohlbefinden des Nutzers verbessern kann.

Es werden zum Beispiel Kühlakkus (z.B. in Form von Kältepacks) mittels eines Verbandes oder einer anderen Stützstruktur im Bereich einer Verletzung am Bein fixiert und gegen diesen gedrückt, um die betreffende Stelle zu kühlen. Es ist zudem bekannt, bei Fieber feuchte Tücher zu verwenden, um dadurch dem Körper Wärme zu entziehen, welche für die Verdunstung des in den feuchten Tüchern enthaltenen Wassers genutzt wird.

Jedoch haben die beschriebenen Maßnahmen zahlreiche Nachteile. Zum Beispiel ist es schwierig, die Kühlakkus oder die feuchten Tücher an einer gewünschten Stelle dauerhaft zu fixieren, d.h., so dass sie bei einer Bewegung des Nutzers nicht verrutschen, und die beschriebenen Lösungen können beim Nutzer Unbehagen auslösen bzw. sind nicht angenehm zu tragen, insb. wenn sich der Nutzer bewegen möchte. Des Weiteren führt die feuchte-Tücher-Lösung dazu, dass nicht nur die umwickelte Körperstellenass ist, sondern auch die Kleidung oder Einrichtungsgegenstände befeuchtet werden.

Als Beispiel für die oben genannte Kühlakku-Lösung offenbart die Offenlegungsschrift DE102006016988A1 eine Fußbekleidung, in die ein Thermoelement zum Entziehen von Wärme integriert ist. Ferner offenbart die UK Patentanmeldung GB2261822A eine Wärmstruktur, insbesondere für einen Ellenbogen, zum Wärmen/Heizen von Körperteilen mittels thermischer Isolierung oder aktiven Heizens des Körperteils bzw. des Ellenbogens. Ferner beschreibt die WO2005/074846A1 ein Personenkühlelement, insbesondere für Patienten, mit mindestens einer Kühlzone, wobei elastische Mittel vorhanden sind, um die Kühlzone gegen die Körperoberfläche einer das Personenkühlelement tragenden Person vorzuspannen, dadurch gekennzeichnet, dass die Kühlzone dreilagig ausgebildet ist mit einer körperzugewandten Innenschicht, einer körperabgewandten Aussenschicht sowie einem dazwischen angeordneten Verdunstungsbereich, wobei die Innenschicht und die Aussenschicht je aus einem wasserdichten und wasserdampfdurchlässigen Material gebildet sind und wobei Wasserzuführmittel vorhanden sind, um den Verdunstungsbereich mit flüssigem Wasser zu versorgen.

### Kurzbeschreibung

In diesem Lichte stellt die vorliegende Erfindung eine Fuß-Kühlmanschette gemäß Anspruch 1 bereit, welche eine oder mehrere der oben genannten Nachteile behebt bzw. mindert. Weitere Ausführungsformen sind in den abhängigen Ansprüchen 2-8 beschrieben. Insbesondere stellt die vorliegende Erfindung eine Fuß-Kühlmanschette bereit, die den Fuß auf einfache aber effektive Weise kühlt, einfach herzustellen ist, praxistauglich ist und zudem einen hohen Tragekomfort hat (z.B. eine Befeuchtung der Umgebung vermeidet/reduziert sowie angenehm und zuverlässig zu tragen ist (selbst wenn der Nutzer sich bewegen möchte)). Die erfindungsgemäße Fuß-Kühlmanschette kann waschbar sein, z.B. bei hohen Temperaturen maschinenwaschbar sein, so dass die Sauberkeit und Hygiene der Fuß-Kühlmanschette gewährleistet ist.

Zum Beispiel kann die erfindungsgemäße Fuß-Kühlmanschette in warmen Sommertagen für ein Wohlbefinden des Nutzers sorgen, d.h. eine Abhilfe bei "glühenden" Füßen schaffen, indem die Fußsohle gekühlt wird, über welche eine große Wärmemenge abgeführt werden kann.

Erfindungsgemäß kann eine Fuß-Kühlmanschette (z.B. Fußumfassungsstruktur, z.B. Kühl-"Schuh") zum Kühlen eines Fußes (insb. eines menschlichen Fußes) gebildet sein aus (z.B. bestehen aus) einem Lagenverbund mit (z.B. bestehend aus) zumindest einer ersten, textilen, saugfähigen, inneren, flexiblen Lage ("saugfähige Textillage") und einer mit dieser zumindest abschnittsweise (mittelbar oder unmittelbar) fest verbundenen (z.B. vernähten) zweiten, äußeren, flexiblen Lage (welche insb, eine Erstreckung hat, die im Wesentlichen gleich oder größer der Erstreckung der inneren/ersten Lage ist), wobei der Lagenverbund, (z.B. insbesondere die innere und/oder die äußere Lage davon) unter Ausbildung/Begünstigung einer dreidimensionalen Struktur zumindest in bestimmten Abschnitten in beständiger Weise vorgeformt ist, welche dreidimensionale Struktur eine Fußaufnahmekavität definiert, die (ausschließlich oder unter anderem) begrenzt ist (insb. nach unten hin) von einem zentralen, länglichen, im Wesentlichen horizontalen Sohlenbereich der Fuß-Kühlmanschette, welcher ausgebildet ist, um die Fußsohle eines Nutzers abzudecken (z.B. im Wesentlichen vollständig oder zumindest überwiegend), sowie einem Rist-und-Spann-Bereich der Fuß-Kühlmanschette (insb. zu den beiden Seiten hin), welcher sich von den beiden Seitenrändern des Sohlenbereichs aus nach oben erstreckt und ausgebildet ist, um den Spann und den Innen- und Außenrist des Nutzers abzudecken (z.B. auch den inneren und den äußeren Fußknöchel), und einem Fersenbereich der Fuß-Kühlmanschette (insb. nach hinten hin), welcher sich von einem hinteren Rand des Sohlenbereichs aus nach oben erstreckt und ausgebildet ist, um die Ferse des Nutzers abzudecken (z.B. die vollständige Ferse oder "Hacke", z.B. auch einen unteren Teil der Achillessehne). Die zweite Lage kann einerseits für das flüssige Kühlmittel im Wesentlichen undurchlässig sein, so dass das in der ersten Lage zwischengespeicherte flüssige Kühlmittel daran gehindert wird bzw. ist, über eine Außenseite der Fuß-Kühlmanschette in flüssiger Form zu entweichen, und andererseits für das in Folge der Wärmezufuhr von dem Fuß (bzw. der Wärmeabfuhr von dem Fuß weg zu dem Kühlmittel) verdampfte Kühlmittel durchlässig sein.

Die erste und die zweite Lage können jeweils gasdurchlässig sein, wobei die (z.B. gesamte) erste Lage ein saugfähiges Textilmaterial (z.B. Wasserabsorptionsmaterial) aufweist (z.B. daraus besteht), so dass die erste Lage vor dem Anlegen der Fuß-Kühlmanschette mit flüssigem Kühlmittel (z.B. Wasser) zum Zweck des Kühlens des Fußes getränkt werden kann, um das Kühlmittel darin zwischen zu speichern und für eine Verdunstung durch eine Wärmeabfuhr von dem Fuß an letzterem bereitzustellen, und wobei die (z.B. gesamte) zweite Lage für flüssiges Kühlmittel (z.B. Wasser) undurchlässig ist (z.B, im Wesentlichen), so dass das in der ersten Lage zwischengespeicherte flüssige Kühlmittel daran gehindert ist, über eine Außenseite der Fuß-Kühlmanschette in flüssiger Form zu entweichen.

Erfindungsgemäß kann die gesamte erste Lage aus demselben Material gebildet sein und/oder kann die gesamte zweite Lage aus demselben Material gebildet sein. Die erste Lage kann ein saugfähiges Wasserabsorptionsmaterial aufweisen oder aus einem solchen bestehen.

Bei dem Kühlmittel kann es sich um Wasser, z.B. körperfremdes Wasser (insbesondere Leitungswasser), einen Alkohol (z.B. Ethanol) oder um eine Wasser-Alkohol-Mischung oder ähnliches handeln.

Erfindungsgemäß ist der Lagenverbund, insbesondere die innere und/oder die äußere Lage davon, unter Ausbildung einer dreidimensionalen Struktur zumindest in bestimmten Abschnitten in beständiger Weise vorgeformt. Der Lagenverbund, insb. die innere und/oder die äußere Lage, unterscheidet sich daher dadurch von einer gewöhnlichen, unbearbeiteten/nicht-vorgeformten Lage, wie z.B. einem herkömmlichen/gewöhnlichen (Hand)-Tuch, dass letztere vollkommen glatt/flach in eine 2-dimensionale Lage (die Dicke des Tuchs vernachlässigend) gestrichen/gezogen werden kann, was mit dem Lagenverbund bzw. der inneren und/oder äußeren Lage aufgrund der beständig vorgeformten Abschnitte nicht möglich ist. Die in Anspruch 1 beschriebene dreidimensionale (Gesamt-)Struktur selbst muss nicht zwingend beständig sein (was der Flexibilität der Lage(n) geschuldet sein kann), in dem Sinne, dass sie z.B. durch ein Zusammenfalten der Fuß-Kühlmanschette (oder ein Herunterklappen des Fersenbereichs auf den Sohienbereich) zwischenzeitlich aus ihrer Form gebracht werden kann. Jedoch kann die in Anspruch 1 beschriebene dreidimensionale (Gesamt-)Struktur, einmal in die oben beschriebene Struktur/Form gebracht, z.B. Bestand haben bzw. in dieser Form bestehen bleiben, sofern die Fuß-Kühlmanschette nicht von Hand bzw. durch äußere Einwirkung aus ihrer Form gebracht wird. Die dreidimensionale Struktur ist dadurch bewirkt bzw. begünstigt, dass zumindest bestimmte Abschnitte des Lagenverbunds, insbesondere der inneren und der äußeren Lage davon, in beständiger Weise vorgeformt sind (d.h., die beständige Vorformung begünstigt (z.B. "erzwingt") die beschriebene dreidimensionale Struktur), z.B. strukturgebend beständig vorbearbeitet sind (z.B. durch Nähen), so dass sie dem Lagenverbund bzw. der inneren und/oder der äußeren Lage abschnittsweise eine 3-dimensionale (Teil-)Struktur verleihen/vorgeben, so dass der Lagenverbund bzw. die jeweilige Lage nicht mehr in eine 2-dimensionale Lage flach/glatt gestrichen/gestrichen werden kann (aufgrund der beständig vorbearbeiteten Abschnitte). Mit anderen Worten kann der Lagenverbund, insbesondere die innere und/oder die äußere Lage davon, z.B. zumindest in bestimmten Abschnitten in beständiger Weise formverfestigt (oder formstabilisiert) sein und/oder in eine beständige Form (z.B. eine beständige Querschnittsform) gebracht sein.

Die Fuß-Kühlmanschette ist also durch ein (Textil-)Formgebungsverfahren (insb. ein Formgebungsverfahren, das aus einer 2-dimensionalen Lage in beständiger Weise eine dreidimensionale Lagenstruktur formt (deren dreidimensionale Form veränderbar sein kann, die aber nicht in die zweidimensionale Lage zurück formbar/streifbar ist) bzw. die 2-dimensionale Lage z.B. durch (teilweises) Trennen und/oder Fügen in eine dreidimensionale Lagenstruktur in beständiger Weise bringt) zumindest in Abschnitten davon beständig vorgeformt, so dass die Fuß-Kühlmanschette (immer wieder) in die in Anspruch 1 beschriebene 3-dimensionale Struktur bringbar ist und die Ausbildung der 3-dimensionalen Struktur durch die beständig vorgeformten Abschnitte begünstigt wird. Aufgrund der Flexibilität der Lagen kann die dreidimensionale Struktur selbst als Ganzes in ihrer Form veränderbar bzw. aus der Form bringbar sein, obwohl der Lagenverband dennoch in den vorgeformten Abschnitten beständig vorgeformt ist.

Zusätzlich oder alternativ kann die erste Lage aus einem Material hergestellt sein oder bestehen, das aus der Gruppe ausgewählt ist, die besteht aus Frottee, Frotteefutter, Tuch, Plüsch, Samt, Velours, Vlies, Baumwolle, Viskose (aus Bambus-Zellstoff), Bambusfasern, Polmaterial (z.B. Polgewebe), Wolle, Wollfutter, Weblammfutter, thermoplastischer Kunststoff (der z.B. eine antibakterielle Wirkung haben kann).

Zusätzlich oder alternativ kann die zweite Lage eine atmungsaktive (gasdurchlässige; für gasförmiges Kühlmittel durchlässige, insbesondere Wasserdampfdurchlässige) und für flüssiges Kühlmittel (z.B. Wasser) undurchlässige Funktionstextilie aufweisen oder daraus bestehen.

Zusätzlich oder alternativ kann der Lagenverbund, insbesondere die innere und/oder die äußere Lage davon, durch Vernähen und/oder Verschweißen und/oder Verkleben von jeweiligen Bereichen davon zumindest in bestimmten Abschnitten in beständiger Weise vorgeformt sein, z.B. durch Überlappungs-Nähen und/oder Überlappungs-Schweißen und/oder Überlappungs-Kleben (d.h., durch Vernähen/Verschweißen/Verkleben zweier sich überlappender Bereiche des Lagenverbunds bzw. der inneren und/oder der äußeren Lage davon. Die beständige Vorformung/strukturgebende Vorbearbeitung bzw. der dadurch beständig vorgeformte/vorbearbeitete Bereich kann also eine Faden-Naht und/oder eine Schweiß-Naht und/oder eine Klebe-Naht aufweisen.

Zusätzlich oder alternativ können die gesamte erste Lage und/oder die gesamte zweite Lage durchgängig und insbesondere monolithisch ausgebildet sein, so dass sie sich durchgängig bzw. monolithisch integral im Sohlenbereich sowie im Rist-und-Spann-Bereich und Fersen-Bereich erstrecken.

Zusätzlich oder alternativ kann die Fuß-Kühlmanschette bzw. deren dreidimensionale Struktur in Längsrichtung vorne offen bzw. kappenfrei ausgebildet sein, so dass der vordere Rand bzw. die Stirnseite des Sohlenbereichs freiliegt bzw. die Fußaufnahmekavität von vorne/stirnseitig frei zugänglich ist, und/oder die Fuß-Kühlmanschette bzw. deren dreidimensionale Struktur kann oben offen bzw. zungenfrei ausgebildet sein, so dass die Fußaufnahmekavität unter Ausbildung einer über dem Sohlenbereich angeordneten Einstiegsöffnung, weiche sich insbesondere entlang der gesamten Längsachse des Sohlenbereichs erstrecken kann, von oben frei zugänglich sein kann bzw. der Rist-und-Spann-Bereich einen ersten/linken und einen zweiten/rechten Flügelabschnitt unterteilt sein kann, die sich von einem jeweiligen Seitenrand des Sohlenbereichs aus nach oben erstrecken können, wobei die beiden Flügelabschnitte insbesondere über den Fersenbereich miteinander verbunden sein können, und/oder die Fuß-Kühlmanschette bzw. deren dreidimensionale Struktur kann im Wesentlichen eine Baggerschaufelstruktur haben.

Zusätzlich oder alternativ können die beiden Flügelabschnitte, insb. deren freien Enden, lösbar aneinander befestigbar sein, z.B. mittels eines Klettverschlusses oder Ösen-Schnür-Systems.

Zusätzlich oder alternativ kann der Lagenverbund, insb. die innere und die äußere Lage davon, dadurch zumindest in bestimmten Abschnitten in beständiger Weise vorgeformt sein, dass zwei zunächst voneinander beabstandete Bereiche des Lagenverbunds, insb. der inneren und/oder der äußeren Lage davon, in Kontakt, z.B. Überlappungskontakt, gebracht und miteinander fest verbunden werden, z.B. miteinander vernäht werden.

Zusätzlich oder alternativ kann ein Bereich zwischen dem jeweiligen Seitenrand des Sohlenbereichs und dem Rist-und-Spann-Bereich in beständiger Weise vorgeformt sein.

Zusätzlich oder alternativ kann der Lagenverbund eine einzelne Tasche oder eine Mehrzahl an Taschen aufweisen, wobei jede Tasche eingerichtet sein kann, um jeweils ein oder mehrere Kühlelemente aufzunehmen. Ein Kühlelement kann somit in der jeweiligen Tasche sicher/zuverlässig angeordnet werden.

Die jeweilige Tasche kann zwischen der ersten und der zweiten Lage ausgebildet sein und z.B. von zumindest einem in beständiger Weise vorgeformten Bereich begrenzt sein. Der in beständiger Weise vorgeformte Bereich dient dann gleichzeitig dazu, die dreidimensionale Struktur zu begünstigen bzw. auszubilden, als auch dazu, die jeweilige Tasche (mit) auszubilden. Die innere/erste Lage schützt zudem vor einem direkten Kontakt mit dem Kühlelement.

Zusätzlich oder alternativ kann in der Fuß-Kühlmanschette zumindest eine Tasche in dem Sohlenbereich der Fuß-Kühlmanschette angeordnet sein und sich entlang eines Längsabschnitts davon erstrecken und/oder zumindest eine Tasche kann in einem medialen Seitenabschnitt des Rist-und-Spann-Bereichs angeordnet sein und kann sich entlang eines Längsabschnitts davon erstrecken und/oder zumindest eine Tasche kann in einem lateralen Seitenabschnitt des Rist-und-Spann-Bereichs sein angeordnet ist und kann sich entlang eines Längsabschnitts davon erstrecken.

Zusätzlich oder alternativ kann sowohl in dem Sohlenbereich als auch in dem medialen Seitenabschnitt des Rist-und-Spann-Bereichs und dem lateralen Seitenabschnitt des Rist-und-Spann-Bereichs (jeweils) eine Tasche angeordnet sein, wobei die Taschen miteinander in Verbindung stehen können und z.B. über eine gemeinsame, insb. verschließbare und öffenbare, Tasche-Öffnung an der Außenseite der Fuß-Kühlmanschette zugänglich sein können, welche sich z.B. entlang zumindest eines Teils des Sohlenbereichs und des Fersenbereichs erstrecken.

Zusätzlich oder alternativ kann die jeweilige Tasche über eine an der Außenseite der Fuß-Kühlmanschette ausgebildete, insb. verschließbare und offenbare, Tasche-Öffnung zugänglich sein, die das jeweilige Innere der Tasche mit der Außenseite der Fuß-Kühlmanschette verbinden kann.

Zusätzlich oder alternativ kann in zumindest einer der Taschen, z.B. in jeder Tasche, ein Kühlelement aufgenommen sein.

Die erste Lage ist die innerste, d.h. die im Gebrauch direkt am Fuß anliegende, Lage des Lagenverbunds und der Fuß-Kühlmanschette.

Zusätzlich oder alternativ kann der Lagenverbund zusätzlich eine dritte, flexible Lage aufweisen, die mit der zweiten Lage zumindest abschnittsweise fest verbunden sein kann und die die Außenfläche der Fuß-Kühlmanschette im Wesentlichen vollständig bilden kann, wobei die dritte Lage durchlässig für gasförmiges Kühlmittel/Wasserdampf und z.B. auch durchlässig für flüssiges Kühlmittel (z.B. Wasser) sein kann. Die dritte Lage kann z.B. als Zierlage und/oder als Schutzlage und/oder als schmutzabweisende Lage ausgebildet sein.

Zusätzlich oder alternativ können gemäß Ausführungsformen der Erfindung alle oder einzelne Lagen des Lagenverbundes (vollständig oder zumindest in Teilbereichen) im direkten und unmittelbaren Kontakt zueinander sein, so dass z.B. die erste Lage direkt und unmittelbar ohne Zwischenschicht/Zwischenlage an die zweite Lage angrenzt und/oder so dass die zweite Lage direkt und unmittelbar ohne Zwischenschicht/Zwischenlage an die dritte Lage angrenzt.

Zusätzlich oder alternativ kann der Lagenverbund, insb. die innere und die äußere Lage davon, dadurch zumindest in bestimmten Abschnitten in beständiger Weise vorgeformt sein, dass ein erster Bereich des Lagenverbunds mit einem zweiten Bereich, der zunächst einen Abstand von dem ersten Bereich haben kann, so fest verbunden sein kann, dass der erste Bereich an den zweiten Bereich entlang einer ersten Linie anstoßen kann, wobei die erste Linie eine Richtungskomponente in Längsrichtung aufweisen kann, die größer sein kann als die Richtungskomponente in Querrichtung, und dass ein dritter Bereich des Lagenverbunds mit einem viertem Bereich, der zunächst einen Abstand von dem dritten Bereich haben kann, so fest verbunden sein kann, dass der dritte Bereich an den vierten Bereich entlang einer zweiten Linie anstoßen kann, wobei die zweite Linie eine Richtungskomponente in Längsrichtung aufweisen kann, die größer sein kann als die Richtungskomponente in Querrichtung, und wobei die erste Linie und die zweite Linie, wenn der Lagenverbund in die dreidimensionale Struktur gebracht ist, zwischen Sohlenbereich und Rist-und-Spann-Bereich angeordnet sein können.

Die erfindungsgemäße Fuß-Kühlmanschette kann z.B. zum Kühlen eines Fußes verwendet werden, indem die erste Lage vor einem Anlegen der Fuß-Kühlmanschette mit flüssigem Kühlmittel (z.B. Wasser (z.B. Leitungswasser)) zum Zweck des Kühlens des Fußes getränkt wird, um das Kühlmittel darin zwischen zu speichern und für eine Verdunstung durch eine Wärmeabfuhr von dem Fuß bereitzustellen, und indem die Fuß-Kühlmanschette anschließend an dem zu kühlenden Fuß angebracht wird (wobei die erste Lage nach innen zeigt und insb. den Fuß des Nutzers kontaktiert). Hierzu kann der Nutzer über die Fußaufnahmekavität in die getränkte/nasse Fuß-Kühlmanschette einsteigen, so dass die Fußsohle des Nutzers auf dem Sohlenbereich steht/angeordnet ist, und die Fuß-Kühlmanschette am Fuß fixieren, z.B. durch ein Verbinden bzw. aneinander Befestigen der beiden Flügelabschnitte. Die zweite Lage verhindert dann ein nach-außen-Dringen des flüssigen Kühlmittels, wodurch einerseits das Kühlmittel für seinen bestimmungsgemäßen Zweck im Inneren der Fuß-Kühlmanschette gehalten wird und wodurch andererseits ein ungewolltes Befeuchten von z.B. einem Teppich verhindert/reduziert wird. Bei Bedarf kann zudem ein Kühlpack in eine dafür vorgesehene Tasche der Fuß-Kühlmanschette gesteckt werden, um den Kühleffekt zu erhöhen. Der Spann-und-Rist-Bereich sowie der Fersenbereich ermöglichen einen zuverlässigen Halt der Fuß-Kühlmanschette an dem Fuß sowie eine großflächige Kühlung des Fußes. Die Verwendung bzw. der Einsatz der flexiblen, saugfähigen Textillage und der flexiblen, zweite Lage ermöglichen zudem einen hohen Tragekomfort und eine relativ einfache Herstellung.

Dementsprechend kann ein Verfahren zum Kühlen eines Fußes nach dem Verdunstungsprinzip unter Verwendung einer Fuß-Kühlmanschette aufweisen Tränken der ersten Lage mit einem flüssigen Kühlmittel, Einbringen eines Fußes in die Fußaufnahmekavität und Kühlen des Fußes durch Verdampfen des von der ersten Lage bereitgestelltem flüssigen Kühlmittels mittels Wärmezufuhr von dem Fuß (bzw. Wärmeabfuhr von dem Fuß hin zu dem flüssigen Kühlmittel). Tränken der ersten Lage kann dabei großflächiges Tränken (z.B. mehr als 75% oder mehr als 50% der Fläche), z.B. vollflächiges Tränken, eines oder mehrerer Bereiche der Fußmanschette (z.B. der im Patentanspruch 1 benannten Bereiche), d.h. z.B. Sohlenbereich, Fersenbereich, Rist-und-Spannbereich, aufweisen. Zum Beispiel kann beim Tränken zumindest der Sohlenbereich großflächig mit Kühlmittel getränkt werden. "Tränken" oder "getränkt" kann hier bedeuten, dass die erste Lage in dem getränkten Bereich (bzw. den Bereichen) vollständig oder nahezu vollständig mit Kühlmittel gesättigt ist, kein oder nahezu kein weiteres Kühlmittel mehr aufnehmen kann. Zum Beispiel kann Tränken eine Sättigung der ersten Lage mit Kühlmittel (z.B. Wasser) von 75% oder mehr, 80% oder mehr, 90% oder mehr, oder 99% oder mehr bedeuten.

Weitere Ausführungsformen der Erfindung sind in den abhängigen Ansprüchen beschrieben, wobei grundsätzlich alle beschriebenen Ausführungsformen miteinander kombinierbar sind und zum Teil Merkmale enthalten, die zur Ausführung der Erfindung nicht zwingend notwendig sind.

### Kurze Beschreibung der Figuren

Die Figuren 1a bis 1d zeigen schematische Seitenansichten der erfindungsgemäßen Fuß-Kühlmanschette.

Die Figur 1e zeigt eine teilweise, schematische Querschnittsansicht der erfindungsgemäßen Fuß-Kühlmanschette entlang der Line 1e-1e in der Figur 1a.

Die Figur 2a zeigt eine schematische Seitenansicht der erfindungsgemäßen Fuß-Kühlmanschette zusätzlich aufweisend Taschen zum Aufnehmen von Kühlelementen und zeigt zusätzlich Kühlelemente.

Die Figur 2b zeigt eine schematische Ansicht von hinten ("auf die Ferse") der erfindungsgemäßen Fuß-Kühlmanschette zusätzlich aufweisend Taschen.

Die Figur 2c zeigt eine schematische Ansicht der erfindungsgemäßen Fuß-Kühlmanschette von unten ("auf die Sohle") zusätzlich aufweisend Taschen sowie eine Verschlusseinrichtung zum Verschließen der Tasche.

Die Figur 2d zeigt eine schematische, teilweise Querschnittansicht der Fuß-Kühlmanschette entlang der Line 2d-2d in der Figur 2a.

Die Figur 3a zeigt zwei Ansichten eines Kühlelements zum Kühlen einer Seite eines Fußes.

Die Figur 3b zeigt zwei Ansichten eines Kühlelements zum Kühlen einer Sohle des Fußes.

Die Figur 4 zeigt eine fotographische Darstellung der erfindungsgemäßen Kühlmanschette mit einer "Baggerschaufelstruktur".

Die Figuren sind nicht notwendigerweise maßstabsgetreu und zeigen eine etwas vereinfachte Darstellung von zahlreichen Merkmalen der Erfindung. Im Hinblick auf die Figuren ist insbesondere zu beachten, dass jedes Merkmal, das in einer Figur gezeigt ist, auch mit einem Merkmal einer Figur, in der dieses Merkmal nicht gezeigt ist, kombiniert werden kann.

### Ausführliche Beschreibung

Es wird nun mit Bezug auf die Figuren die erfindungsgemäße Fuß-Kühlmanschette 10 zum Kühlen eines Fußes beschrieben. Es kann sich bei dem Fuß um einen menschlichen Fuß handeln. Die Bezeichnung "Fuß" kann hierin den eigentlichen Fuß (pes), das Sprunggelenk bzw. den Knöchel (malleolus) und den Unterschenkel (crus), z.B. einen unteren, an den Knöchel angrenzenden Abschnitt davon, einschließen oder sie kann nur den eigentlichen Fuß und das Sprunggelenk bzw. den Knöchel einschließen oder sie kann nur den eigentlichen Fuß einschließen.

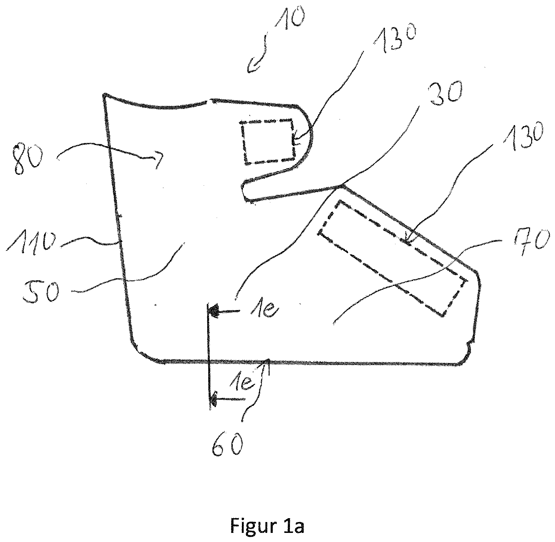

Die Figur 1a zeigt eine Seitenansicht der Fuß-Kühlmanschette 10.

Die Fuß-Kühlmanschette 10 kann aus einem Lagenverbund 30 gebildet sein oder aus einem solchen bestehen. Der Lagenverbund 30 kann zumindest zwei flexible Lagen bzw. Schichten aufweisen, die zumindest abschnittsweise fest miteinander verbunden sind und unten näher beschrieben werden.

Der Lagenverbund 30 kann beständig zumindest in Abschnitten davon so vorgeformt sein, dass er bzw. die Fuß-Kühlmanschette 10 in eine dreidimensionale Struktur bringbar ist, welche von den vorgeformten Abschnitten begünstigt ist. Das bedeutet, dass der Lagenverbund so in Abschnitten davon vorgeformt sein kann, dass diese abschnittsweise Vorformung (z.B. strukturgebende Vorbearbeitung) zwar permanent in den Lagenverbund 30 eingebracht ist und grundsätzlich permanent erhalten bleibt (insb. unter Ausbildung von einem jeweiligen dreidimensionalen, nicht 2-dimensional glatt-streifbaren Teil-Bereich), der Lagenverbund 30 selbst als Ganzes aber immer noch flexibel und verformbar ist, und deswegen auch die dreidimensionale Struktur aus der Form bringbar ist.

In anderen Worten kann die Vorformung des Lagenverbunds 30 in bestimmten Abschnitten dem Lagenverbund 30 bzw. der Fuß-Kühlmanschette 10 eine Tendenz geben, die dreidimensionale Struktur anzunehmen.

Der Lagenverbund kann so in Abschnitten davon vorgeformt sein, dass die dreidimensionale Struktur zu einem (z.B. menschlichen) Fuß korrespondiert bzw. an eine Fußform angepasst ist.

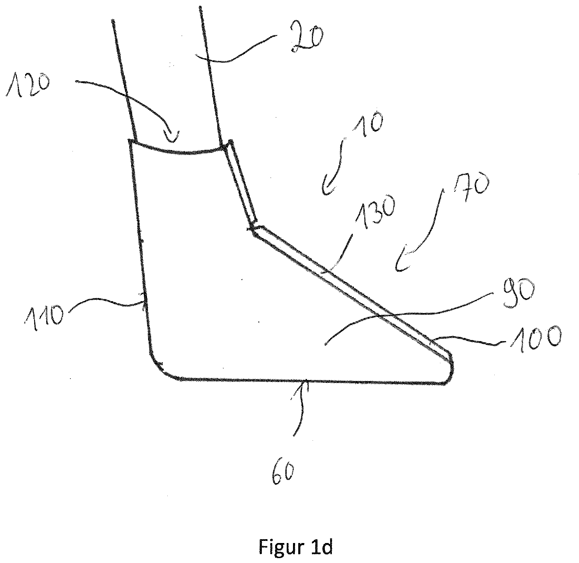

Diesbezüglich kann der Lagenverbund 30 so vorgeformt sein, dass er einen Sohlebereich 60 zum zumindest teilweisen, z.B. vollständigen, Unterlappen bzw. Bedecken bzw. Abdecken der Fußsohle eines Fußes 20 (Fig. 1d) ausbildet bzw. aufweist.

Der Sohlenbereich 60 kann eine solche Form und solche Abmessungen aufweisen, dass er zur Form bzw. zu den Abmessungen einer Fußsohle eines (z.B. menschlichen) Fußes korrespondiert.

Der Sohlenbereich 60 kann sich im Wesentlichen parallel zur Fußsohle (d.h. bei einem stehenden Menschen horizontal) erstrecken.

Die Lagenverbund 30 kann ferner in bestimmten Abschnitten so vorgeformt sein, dass er zusätzlich einen Rist-und-Spann-Bereich 70 ausbildet, der den Spann sowie den Innen- und den Außenrist (bzw. die mediale und die laterale Seitenfläche) des Fußes 20 überlappt bzw. bedeckt bzw. abdeckt.

Der Rist-und-Spannbereich 70 kann sich von den beiden Seitenrändern (z.B. der medialen und der lateralen Seite) des Sohlenbereichs 60 aus in proximaler Richtung zum Körper ("nach oben") erstrecken.

Der Rist-und-Spann-Bereich 70 kann eine Form und Abmessungen aufweisen, die zu der Form bzw. zu den Abmessungen des Innenrist, des Außenrists, der Ferse und des Spanns eines (z.B. menschlichen) Fußes korrespondieren.

Beispielsweise kann der Lagenverbund 30 beständig in Abschnitten bzw. Bereichen davon vorgeformt werden, indem ein erster Bereich des Lagenverbunds 30 mit einem zweiten Bereich, der zunächst einen Abstand von dem ersten Bereich hat, so verbunden ist, dass der erste Bereich an den zweiten Bereich entlang einer ersten Linie anstößt, wobei die erste Linie eine Richtungskomponente in Längsrichtung aufweist, die größer ist als die Richtungskomponente in Querrichtung, und indem ein dritter Bereich des Lagenverbunds mit einem viertem Bereich, der zunächst einen Abstand von dem dritten Bereich hat, so verbunden ist, dass der dritte Bereich an den vierten Bereich entlang einer zweiten Linie anstößt, wobei die zweite Linie eine Richtungskomponente in Längsrichtung aufweist, die größer ist als die Richtungskomponente in Querrichtung, und wobei die erste Linie und die zweite Linie, wenn der Lagenverbund 30 die dreidimensionale Struktur annimmt, zwischen Sohlenbereich 60 und Rist-und-Spann-Bereich 70 angeordnet sind, d.h. z.B. die "Grenze" zwischen Sohlenbereich 60 und Rist-und-Spann-Bereich 70 bildet. Das Verbinden kann dabei z.B. durch Vernähen und/oder Verkleben erfolgen und eventuell überschüssiges Material, das im Bereich der Verbindungslinien einen Wulst bildet, kann z.B. mittels Abschneidens entfernt werden.

Der Rist-und-Spann-Bereich 70 kann sich in proximaler Richtung entlang des Beines (d.h. in vertikaler Richtung bei einem stehenden Menschen) nur bis etwa zum unteren (d.h. Fußseitigen) Rand des Knöchels erstrecken, ohne an diesen anzustoßen.

Die Rist-und-Spann-Bereich 70 kann sich auch, wie es in der Figur 1a gezeigt ist, entlang des Unterschenkel zum Körper hin bis über den Knöchel hinaus erstrecken, um der Fuß-Kühlmanschette 10 eine höhere Stabilität und einen besseren Halt zu geben und/oder um eine Kühlung des Knöchels/Sprunggelenks zu ermöglichen.

Der Lagenverbund 30 kann ferner in Abschnitten davon so vorgeformt sein, dass die dreidimensionale Struktur einen Fersenbereich 110 ausbilden kann, der sich von einem hinteren Rand (z.B. Rückseite-Randbereich) des Sohlenabschnitts 60 aus nach oben erstrecken kann, um die Ferse des Fußes 20 des Benutzers abzudecken bzw. zu bedecken.

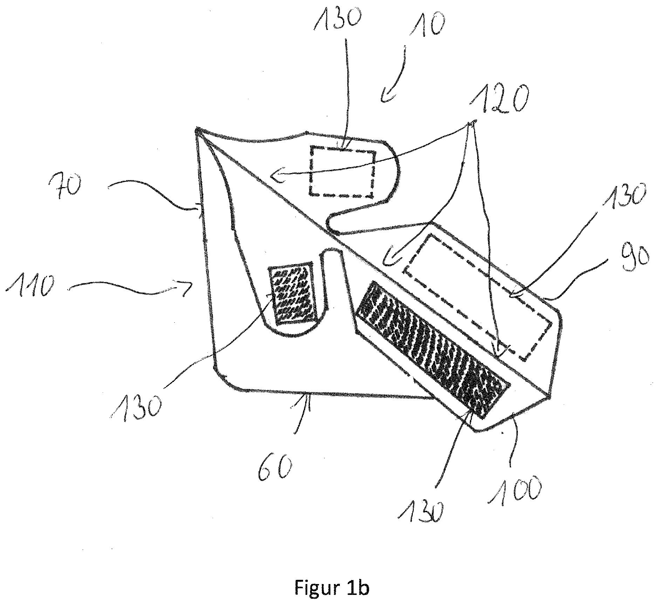

Mit Bezug auf Figur 1b kann der Rist-und-Spann-Bereich 70 so gebildet sein, dass er einen ersten Flügelabschnitt 90 und einen zweiten Flügelabschnitt 100 ausbildet, wobei sich ein jeder der Flügelabschnitte 90, 100 von einem gegenüberliegenden Seitenbereich (d.h. dem medialen oder lateralen Seitenbereich bzw. Seitenrand) des Sohlenbereichs 60 proximal zum Körper, d.h. nach "oben" erstreckt.

Der erste 90 und der zweite Flügelabschnitt 100 können sich in Längsrichtung des Sohlenbereichs 60 (d.h. in Längsrichtung des Fußes 20) von der Ferse bis zum gegenüberliegenden Längsende des Sohlenbereichs 60 erstrecken.

Das Längsende des Sohlenbereichs 60 kann dabei so gebildet sein, dass der Sohlenbereich 60 die Fußsohle vollständig unterlappt oder nur teilweise unterlappt. Zum Beispiel kann sich der Sohlebereich 60 nur von der Ferse des Fußes bis zum Ansatz der Zehen erstrecken, so dass der Sohlebereich 60 die Zehen selbst nicht unterlappt.

Der erste 90 und der zweite 100 Flügelabschnitt können so gebildet sein, dass sie mittels des Fersenbereichs 110, der bei angezogener Fuß-Kühlmanschette 10 zu dem Fersenbereichs des Fußes 20 korrespondiert und diesen überlappt bzw. abdeckt, miteinander verbunden sind.

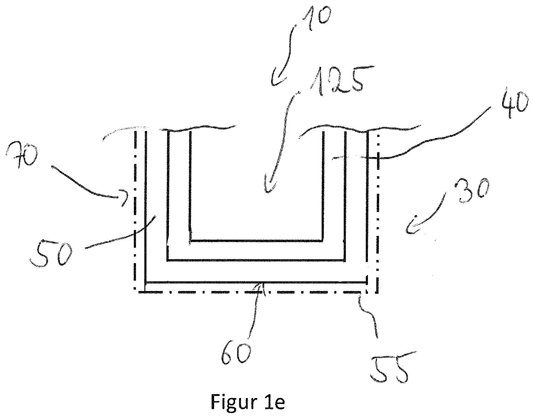

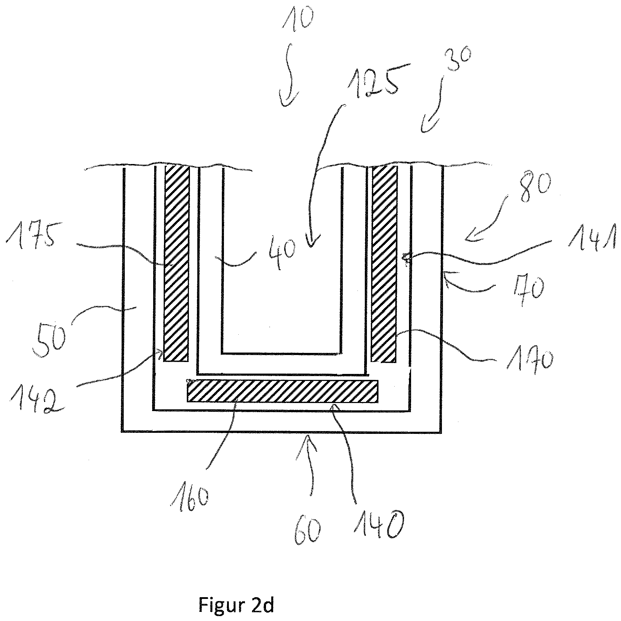

Der Lagenverbund 30, der zumindest in bestimmten Abschnitten davon beständig vorgeformt (z.B. bearbeitet) ist, kann so vorgeformt sein, dass die dreidimensionale Struktur, die aufgrund der Vorformung/Bearbeitung erhalten werden kann, eine Fußaufnahmekavität 125 (siehe Figur 1e oder Figur 2d) definiert, in die der Fuß 20 des Benutzers über eine Einstiegöffnung 120 eingebracht werden kann.

Die Fußaufnahmekavität 125 kann von dem Rist-und-Spann-Bereich 70, dem Sohlenbereich 60 und dem Fersenbereich 110 begrenzt, z.B. zumindest teilweise umschlossen/abgedeckt sein.

Wie erwähnt, kann die Fuß-Kühlmanschette 10 kann eine Einstiegsöffnung 120 zum Einbringen des Fußes in die Fuß-Kühlmanschette 10 aufweisen.

Die Einstiegsöffnung 120 kann mittels des ersten 90 und des zweiten 100 Flügelabschnitts (und z.B. mittels des Fersenabschnitts 110) definiert sein und kann sich von dem Fersenbereich 110 durchgängig und vollständig im Rist-und-Spann-Bereich 70 bis zu einem (bezogen auf den Fuß) vorderen Längsende des Rist-und-Spann-Bereichs 70 erstrecken. In anderen Worten kann die Kühl-Fußmanschette 10 zungenfrei sein bzw. kann keine Zungen aufweisen und/oder kann Kappen-frei sein bzw. keine Kappe aufweisen.

Das vordere Längsende des Rist-und-Spann-Bereichs 70 kann zu dem vorderen Längsende des Sohlenbereichs 60 korrespondieren, so dass beide vorderen Längsenden in derselben Ebene, die von der Längsrichtung der Sohle in Normelenrichtung geschnitten wird, definiert sind.

Die oben-genannte Ebene, und damit die vorderen Längsenden von Rist-und-Spann-Bereich 70 und Sohlenbereich 60, kann so gewählt sein, dass die Zehen eines Fußes freiliegen, oder kann so gewählt sein, dass die Zehen (z.B. vollständig) über- bzw. unterlappt werden.

Alternativ (nicht gezeigt) kann die Einstiegsöffnung 120, ähnlich wie bei einem konventionellen Stiefel oder Schuh, auch nur entlang eines Abschnitts der Oberseite des Riss-und-Spann-Bereichs 70 in Längsrichtung der Fuß-Kühlmanschette 10 gebildet sein, so dass die Fuß-Kühlmanschette 10 bzw. die Fußaufnahmekavität 125 über den Fuß 20 gezogen werden kann, um den Fuß 20 zu umhüllen.

Wenn die Zehen über- bzw. unterlappt werden, können die Zehen dennoch in Längsrichtung des Fußes "nach vorne" freiliegen, d.h. die Fuß-Kühlmanschette 10 kann in Längsrichtung vorne offen ausgebildet sein. Eine solche Öffnung und/oder das Freiliegen der Zehen kann den Luftstrom zwischen dem Inneneren der Fuß-Kühlmanschette 10 und der Umgebung verbessern und kann, wie unten näher beschrieben ist, die Verdunstung von Kühlmittel und damit die Kühlung des Fußes verbessern.

Die Einstiegsöffnung 120 kann mittels einer Einstiegsöffnungs-Verschlusseinrichtung 130 zumindest teilweise verschließbar und öffenbar sein, um die Fuß-Kühlmanschette 10 am Fuß zu fixieren bzw. von diesem zu entfernen.

Gemäß den Figuren ist die Einstiegsöffnungs-Verschlusseinrichtung 130 mittels Klettverschlussteilen gebildet, die zueinander korrespondierend an dem ersten 90 und dem zweiten 100 Flügelabschnitt gebildet sind (und/oder dort befestigt sind), um die beiden Flügelabschnitte 90 und 100 zum Verschließen der Einstiegsöffnung 120 aneinander zu befestigen. Obwohl in den Figuren zwei Paare von korrespondierenden Klettverschlussteilen gezeigt sind, kann die Einstiegsöffnungs-Verschlusseinrichtung 130 auch nur ein Paar aufweisen oder kann mehr als zwei Paare aufweisen.

In diesem Zusammenhang zeigt die Figur 1b eine schematische, perspektivische Ansicht des ersten und des zweiten Flügelabschnitts 90, 100 und der Einstiegsöffnung-Verschlusseinrichtung 130, die als zwei Paare von korrespondierenden Klettverschlussteilen 130 ausgebildet ist, wobei jedes Paar einen "männlichen" und einen "weiblichen" Klettverschlussteil aufweist, die aneinander befestigbar und voneinander lösbar sind, um die Einstiegsöffnung 120 zu öffnen oder zu verschließen, indem der erste 90 und der zweite 100 Flügelabschnitt teilweise überlappt werden und die männlichen und weiblichen Klettverschlussteile durch Andrücken miteinander verbunden werden.

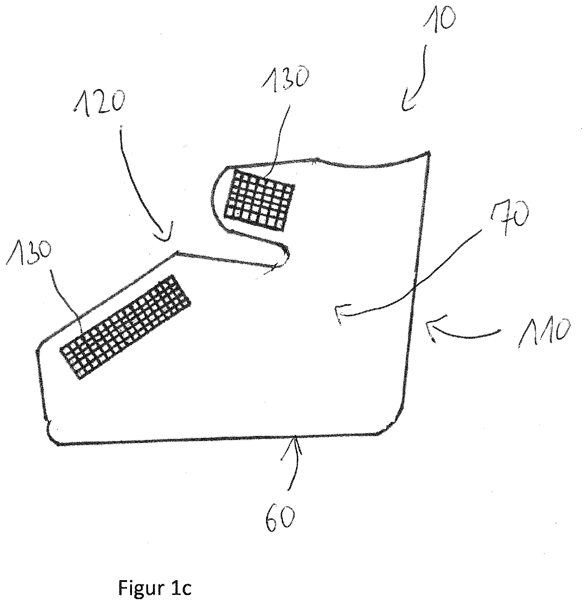

Diesbezüglich zeigt die Figur 1c zeigt eine Ansicht von der Seite der Fuß-Kühlmanschette 10, die der in der Figur 1a gezeigten Seite gegenüberliegt, und zeigt ein erstes, z.B. männliches oder weibliches, Klettverschlussteil 130, und die Figur 1a zeigt ein dazu korrespondierendes, weibliches bzw. männliches Klettverschlussteil 130, das sich auf der hinteren Seite des in der Figur 1a dargestellten Flügelabschnitts befindet und deshalb schematisch mit einer Strich-Linie angedeutet ist.

Alternativ oder zusätzlich kann die Einstiegsöffnungs-Verschlusseinrichtung 130 auch mittels Ösen und Schnur, mittels Schließen, mittels Reisverschluss, mittels Clips, mittels Haken und Ösen, mittels Klebestreifen, mittels Druckknopf, mittels Knopf und korrespondierendem Knopfloch oder auf andere Art gebildet sein.

Die Figur 1d zeigt eine Fuß-Kühlmanschette 10 die am Fuß 20 eines Menschen fixiert ist, indem die Einstiegsöffnung 120 mittels der Einstiegsöffnung-Verschlusseinrichtung 130 teilweise so verschlossen ist, dass die verbleibende, offene Einstiegsöffnung 120 im Wesentlichen dem Durchmesser des Fußes (in der Figur 1d des Unterschenkels) entspricht. Die Figur 1e zeigt eine schematische partielle Querschnittsansicht der Fuß-Kühlmanschette 10 entlang der Linie 1e-1e in der Figur 1a. Anhand der Figur 1e wird im Folgenden der Lagenverbund 30 und der Beitrag davon zur Kühlung des Fußes 20 beschrieben.

Der Lagenverbund 30 der Fuß-Kühlmanschette 10 kann zumindest eine erste Lage 40 und eine zweite Lage 50 aufweisen (oder aus diesen bestehen), die zumindest abschnittsweise (z.B. an den Rändern der ersten 40 und der zweiten Lage 50 und/oder in einem oder mehreren Bereichen, die von den Rändern entfernt sind) oder vollständig fest miteinander verbunden sind, z.B. durch Vernähen und/oder Verkleben.

Die erste Lage 40 ist erfindungsgemäß dabei die innere Lage, d.h. die Lage, die sich bei der Benutzung der Fuß-Kühlmanschette 10 näher am Fuß 20 befindet. Die zweite Lage 50 kann die äußere Lage sein, d.h. die Lage, die sich bei der Benutzung der Fuß-Kühlmanschette 10 weiter vom Fuß 20 entfernt befindet.

In anderen Worten kann die erste Lage 40 den Fuß 20 umhüllen, während die zweite Lage 50 die erste Lage 40 umhüllt.

Die innere Lage 40 ist erfindungsgemäß die innerste, d.h. am nächsten am Fuß 20 liegende, Lage des Lagenverbundes 30 und der Fuß-Kühlmanschette 10. Die erste Lage 40 und die zweite Lage 50 können beide flexibel bzw. plastisch verformbar sein, so dass auch der Lagenverbund 30 flexibel ist, wobei der Lagenverbund 30 dennoch in die beständige, dreidimensionale Struktur vorgeformt sein kann (siehe oben).

Die erste Lage 40 kann eine einteilige Lage sein, d.h. sie kann aus einem Stück bzw. monolithisch integral gebildet sein und sich durchgängig erstrecken. Zum Beispiel kann die erste Lage 40 aus einem einzelnen, zusammenhängenden Stück Stoff (z.B. Tuch) gebildet sein.

Die zweite Lage 50 kann eine einteilige Lage sein, d.h. sie kann aus einem Stück bzw. monolithisch integral gebildet sein und sich durchgängig erstrecken. Zum Beispiel kann die zweite Lage 50 aus einem einzelnen, zusammenhängenden Stück Stoff (z.B. Tuch) gebildet sein.

Dementsprechend können sich der Sohlenbereich 60, der Fersenbereich 110 und der Riss-und-Spann-Bereich 70, die mittels des Lagenverbundes 30, der z.B. die erste und die zweite Lage 40, 50 aufweist, gebildet sind, monolithisch integral erstrecken. Das heißt, der Lagenverbund 30, kann bezogen auf die Richtung, die quer zur Stapelrichtung der einzelnen Lagen ist, vollständig einstückig sein.

Die erste Lage 40 kann dazu eingerichtet sein, flüssiges Kühlmittel (z.B. Wasser) aufzunehmen, z.B. durch Absorption und/oder durch Adsorption, zu speichern, und das Kühlmittel (z.B. in der Gas- bzw. Dampf-Phase davon) wieder abzugeben.

Die erste Lage 40 kann eine gasdurchlässige (z.B. luftdurchlässige), saugfähige bzw. Wasserspeicherfähige Lage sein, die zum Beispiel aus einem Kühlmittel-saugfähigen/Kühlmittelspeicherfähigen (z.B. Wasser-saugfähigen/Wasser-speicherfähigen Material), z.B. einem textilen Material, besteht oder ein solches aufweist.

Die erste Lage 40 kann z.B. aus Naturfasern, z.B. aus pflanzlichen Naturfasern, z.B. aus Baumwolle und/oder Leinen bestehen oder solche Fasern aufweisen.

Die Fasern können zur Bildung der ersten Lage 50 gewirkt, gewebt, gestrickt und/oder auf andere Art und Weise kombiniert sein

Die erste Lage 40 kann z.B. ein textiles Gewebe, z.B. insbesondere Frottee (z.B. Baumwoll-Frottee oder Leinen-Frottee), aufweisen oder daraus bestehen.

Zusätzlich oder alternativ kann die erste Lage 40 atmungsaktiven Schaumstoff, Schaumgummi, Latexschaum, Kaltschaummaterial und/oder Weichschaum, ein Polstermaterial (z.B. Weichpolstermaterial; z.B. Polyethylen, Copolymeren, Polyurethan und/oder Dyatec) aufweisen oder daraus bestehen.

Die erste Lage 40 kann eine innere (z.B. innerste) Fläche bzw. Fuß-Kontaktfläche der Fuß-Kühlmanschette 10 im Wesentlichen vollständig oder vollständig bilden. In anderen Worten kann die erste Lage 40 eine Begrenzung der Fußkavität 125 definieren.

Die zweite (äußere) Lage 50 kann eine gasdurchlässige (z.B. luftdurchlässige) und für flüssiges Kühlmittel undurchlässige (z.B. wasserundurchlässige) Lage sein.

Als zweite Lage 50 kann zum Beispiel eine wasserdichte und/oder wasserabweisende Textilschicht, die gasdurchlässig ist, verwendet werden. Die zweite Lage 50 kann z.B. aus Kunstfasern (z.B. zellulosische Fasern, z.B. Kunststoff (PS, PET, PA, PPS, Aramid, PAN, PE, PVC, PTFE, etc.) bestehen oder solche aufweisen, die gewirkt, gewebt, gestrickt und/oder auf andere Art und Weise kombiniert sein können. Zum Beispiel kann die zweite Textilschicht aus Gore-Tex, Sympatex oder einem ähnlichen "Funktionsmaterial" bzw. einer "Funktionstextilie", die im Wesentlichen wasserundurchlässig und atmungsaktiv (gasdurchlässig) ist, bestehen. Ein solches Material/Textile kann insbesondere geruchfrei, antibakteriell, schmutzabweisend, thermoregulierend, antimikrobiell, elastisch, strapazierfähig, pflegeleicht und/oder winddicht sein. Alternativ oder zusätzlich kann ein solches Material/Textil insbesondere auch UV-Schutz für die darunter liegenden Lagen bieten.

Die zweite Lage 50 kann eine Außenfläche 80 der Fuß-Kühlmanschette 10 vollständig oder im Wesentlichen vollständig bilden.

Der Lagenverbund 30 kann zusätzlich eine flexible dritte Lage 55 aufweisen, die in der Figur 1e schematisch mittels einer Strich-Punkt-Punkt-Linie gezeigt ist. Die dritte Lage 55 kann gasdurchlässig und/oder schmutzabweisend (z.B. mittels einer schmutzabweisenden Beschichtung) sein. Die dritte Lage 55 kann aus einem Material bestehen, das der Fuß-KühlManschette 10 eine ansprechende ästhetische Anmutung gibt.

Die dritte Lage 55 kann z.B. Polyethylen, Copolymere, Polyurethan und/oder Dyatec aufweisen oder daraus bestehen.

In Ausführungsformen der Erfindung kann optional zwischen der ersten 40 und der zweiten Lage 50 und/oder zwischen der zweiten Lage 50 und der dritten Lage 55 ein leichtes und luftdurchlässiges Abstandsmaterial angeordnet sein, das zum Beispiel von der Firma Otto Stockmayer + Sohn GmbH, Pirmasens, Deutschland erhältlich ist.

Die dritte Lage 55 kann die zweite Lage 50 auf der Außenseite davon vollständig oder nur teilweise umhüllen und kann dazu mit der ersten 40 und/oder mit der zweiten 50 Lage verbunden sein, z.B. mittels Vernähens, Klebens und/oder auf andere Art und Weise.

In dem Fall, dass eine dritte Lage 55 bereitgestellt ist, kann die dritte Lage 55 einen Teil oder die gesamte Außenfläche 80 der Fuß-Kühlmanschette 10 bilden.

Für die dritte Lage 55 kann z.B. ein Material, wie es für die erste oder die zweite Lage 40, 50 vorgesehen ist, verwendet werden. Für die dritte Lage 55 kann ein Material mit guten thermischen Isolationseigenschaften wie Wolle, Styropor oder ähnliches verwendet werden und/oder ein Material, das gegen Abrieb und Verschmutzung widerstandsfähig ist, wie z.B. Aramid, Nylon etc. Die dritte Lage 55 kann kühlmittelundurchlässig (z.B. wasserundurchlässig) sein oder sie kann kühlmitteldurchlässig (z.B. wasserdurchlässig) sein.

In den folgenden Absätzen wird beschrieben, wie in der ersten Lage 40 gespeichertes Kühlmittel den Fuß 20 kühlen kann, wobei exemplarisch Wasser als Kühlmittel verwendet wird. Zur Kühlung können dabei einer oder mehrere Faktoren beitragen.

Wenn das Wasser, das in der ersten Lage 40 gespeichert ist, einen niedrigere Temperatur hat als der Fuß 20, kann Wärme von dem Fuß 20 gemäß dem Temperaturgradienten zu dem Wasser übertragen werden, wodurch die Temperatur des Fußes 20 verringert wird und die Temperatur des Wassers ansteigt.

Ferner kann das Wasser der Lage 40 verdunsten/verdampfen (d.h. von der flüssigen in die Gasphase übergehen), wodurch dem Fuß Wärme entzogen wird, was zu einer Verringerung der Fuß-Temperatur führt. Da die Verdunstung des Wassers umso effizienter ist, je niedriger der Wasser(dampf)-Partialdruck in der Umgebung ist, findet die Verdunstung effizienter statt, wenn die Konzentration des schon verdunsteten Wassers in der Luft, d.h. der Wasserdampfpartialdruck, verringert wird.

Dies kann dadurch geschehen, dass trockenere Luft (d.h. mit einem geringeren Wasserdampfpartialdruck) von der Umgebung über die zweite Lage 50 (die gasdurchlässig ist) und/oder die erste Lage 40 (die ebenfalls gasdurchlässig ist) zugeführt wird, und/oder dadurch, dass der Wasserdampf über die erste 40 und/oder die zweite Lage 50 der Umgebung zugeführt wird. Das Zuführen kann z.B. durch Diffusion der jeweiligen Moleküle in der Gasphase geschehen.

Beide Mechanismen der Verringerung des Wasserdampfpartialdrucks können alleine oder gleichzeitig auftreten, und in beiden Fällen sinkt der Wasserdampfpartialdruck in einem Bereich der ersten Lage 40 ab, wodurch die Verdunstung des Wassers und folglich die Kühlung des Fußes effizienter stattfinden kann.

Die Kühlung kann ebenfalls verbessert sein, wenn, wie oben beschrieben, die Zehen eines Fußes 20, oder zumindest die Vorderseite davon, zur Umgebung freiliegen, so dass Luft über die Zehen in die Fuß-Kühlmanschette 10 gelangen kann bzw. Wasserdampf in die Umgebung abgeführt werden kann.

Sofern ein anderes Kühlmittel als Wasser verwendet wird, funktioniert die Kühlung nach dem gleichen Prinzip, wobei dem Fuß mittels der Überführung des Kühlmittels in die Gasphase Wärme entzogen wird. Die erste Lage 40 kann dann mit einem Material bereitgestellt sein, dass zum Zwischenspeichern des jeweiligen Kühlmittels geeignet ist, und die zweite Lage 50 kann mit einem Material bereitgestellt sein, das für dieses Kühlmittel in der flüssigen Phase davon undurchlässig ist, aber das Kühlmittel in der Gasphase davon passieren lässt.

Da die zweite Lage 50 im Wesentlichen undurchlässig für flüssiges Kühlmittel (z.B. wasserundurchlässig) ist und die erste Lage 40 im Wesentlichen vollständig umhüllt, kann vermieden werden, dass die Umgebung der Fuß-Kühlmanschette 10 mit dem in der ersten Lage 40 gespeicherten flüssigen Kühlmittel (z.B. Wasser) in Berührung kommt.

Dadurch kann vermieden werden, dass die Kleidung oder die Einrichtungsgegenstände (z.B. ein Bett oder Sofa oder Teppich) eines Benutzers bei der Benutzung der Fuß-Kühlmanschette 10 mit dem flüssigen Kühlmittel in Kontakt kommen bzw. nass werden.

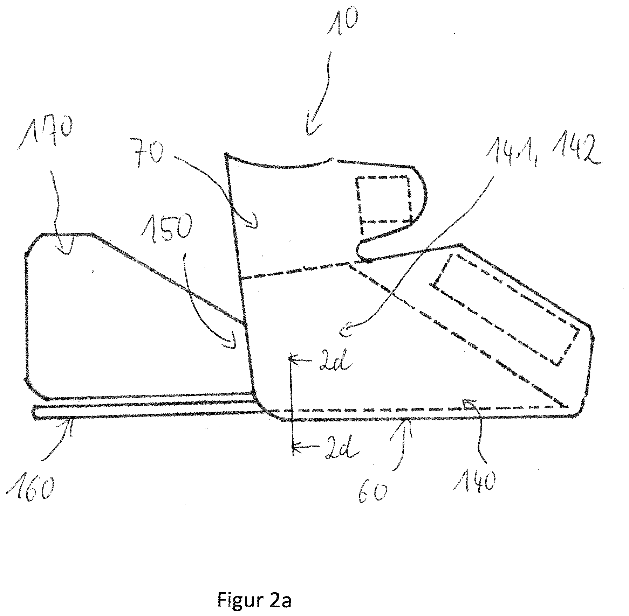

Mit Bezug auf die Figuren 2a bis 2e kann die Fuß-Kühlmanschette 10 bzw. der Lagenverbund 30 zusätzlich eine oder mehrere Tasche 140, 141, 142 zum Aufnehmen von jeweils einem oder mehreren Kühlelementen 160, 170, 175 aufweisen.

Ein Kühlelement kann z.B. ein Kälteakku, ein Kältepack, Eis, Peltier/Seebeck-Element(e), etc. sein oder kann allgemein jede Einrichtung sein, die ihrer Umgebung Wärme entziehen kann. Vorteilhafterweise hat das Kühlelement dazu eine Oberflächentemperatur, die unter derjenigen eines zu kühlenden Fußes liegt.

Die eine Tasche 140, 141, 142 oder die Mehrzahl an Taschen können zwischen der ersten 40 und der zweiten Lage 50 gebildet sein.

Eine Tasche 140 kann so angeordnet sein, dass sie sich im Sohlenbereich 60 erstreckt und im Wesentlichen parallel dazu erstreckt und sich in einem Teil des Sohlenbereichs 60 oder im Wesentlichen über den ganzen Sohlenbereich 60 erstreckt.

Eine Tasche 141 kann zusätzlich oder alternativ so angeordnet sein, dass sie sich in einem (auf den Fuß bezogen) lateralen Seitenabschnitt des Rist-und-Spann-Bereichs 70 erstreckt. Die Tasche 141 kann sich in Längsrichtung des Rist-und-Spann-Bereichs 70 erstrecken und kann sich im Wesentlichen vollständig oder teilweise über den lateralen Seitenabschnitt des Rist-und-Spann-Bereichs 70 erstrecken.

Eine Tasche 142 kann zusätzlich oder alternativ so angeordnet sein, dass sie sich in einem (auf den Fuß bezogen) medialen Seitenabschnitt des Rist-und-Spann-Bereichs 70 erstreckt. Die Tasche 142 kann sich in Längsrichtung des Rist-und-Spann-Bereichs 70 erstrecken und kann sich im Wesentlichen vollständig oder teilweise über den medialen Seitenabschnitt des Rist-und-Spann-Bereichs 70 erstrecken.

Die Taschen 140, 141, 142 können separat voneinander sein, d.h. sie können jeweils einen voneinander getrennten Innenraum haben, oder sie können so gebildet sein, dass ihre jeweiligen Innenräume miteinander verbunden sind und einen gemeinsamen Innenraum bilden.

Jede Tasche kann eine separate Taschen-Öffnung aufweisen, die in der Außenfläche 80 der Fuß-Kühlmanschette 10 gebildet ist und über die ein oder mehrere Kühlelemente in die Tasche einbringbar sind.

Wie es in den Figuren 2a bis 2c dargestellt ist, kann eine Tasche-Öffnung 150 in der Außenfläche 80 gebildet sein, über die gleichzeitig auf mehrere, z.B. alle (hier: drei), in der Fuß-Kühlmanschette 10 bereitgestellte Taschen 140, 141, 142, die miteinander in Verbindung stehen, zugreifbar ist.

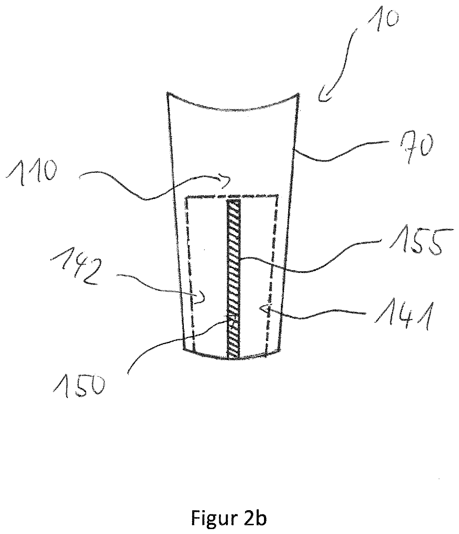

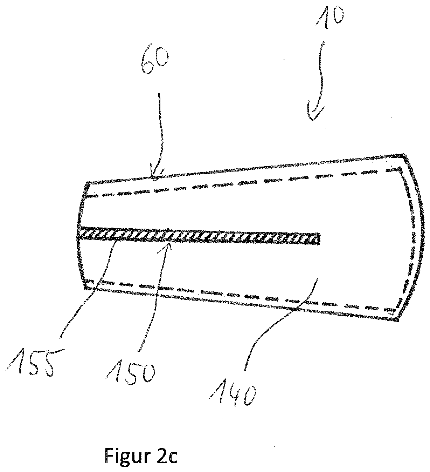

Gemäß den Figuren 2b und 2c, die eine Ansicht "von hinten" auf den Fersenbereich 110 der Fußkühlmanschette 10 bzw. eine Ansicht "von unten" auf den Sohlebereich 60 der Fuß-Kühlmanschette 10 zeigen, erstreckt sich die Tasche-Öffnung 150 von dem Ferse-Bereich 110 vertikal nach unten bzw. in distaler Richtung, erstreckt sich ununterbrochen um die Ferse herum in den Sohlenabschnitt 60 und erstreckt sich weiter in Längsrichtung des Sohlenabschnitts 60. Wie in der Figur 2c ersichtlich, muss sich die Tasche-Öffnung 150 nicht vollständig bis zu dem Zehen-seitigen Längsrand der Tasche 140 erstrecken.

Die Tasche-Öffnung 150 kann mittels einer Tasche-Öffnung-Verschlusseinrichtung 155 öffenbar und verschließbar sein, so dass eine Benutzter die Tasche-Öffnung 150 öffnen bzw. verschließen kann. Die Tasche-Öffnung-Verschlusseinrichtung 155 kann, wie die Einstiegsöffnung-Verschlusseinrichtung 130, z.B. einen Reisverschluss (in den Figuren 2a bis 2c dargestellt), Ösen und Schnur, Klettverschluss, Klebestreifen, Schließen, Löcher und Knöpfe, Clips und/oder ähnliches aufweisen.

In den Figuren 2a, 2b und 2c ist ein Umfangsrand/Umrandung der miteinander in Verbindung stehenden Taschen 140, 141, 142 bzw. des gemeinsamen Innenraums davon schematisch mit einer Unterbrochener-Strich-Linie dargestellt.

Die Figur 2a zeigt die Fuß-Kühlmanschette 10 und zwei Kühlelemente 160, 170 (ein drittes Kühlelement 175 (siehe Fig. 2d) ist nicht sichtbar, da es in dieser Ansicht vom zweiten Kühlelement 170 verdeckt ist), die über die Tasche-Öffnung 150 teilweise in die Taschen 140 (Kühlelement 160), 141 (Kühlelement 170) und 142 (Kühlelement 175, nicht sichtbar) eingebracht bzw. aus diesen entfernt sind.

Die Figur 2d zeigt eine schematische partielle Querschnittsansicht der Fuß-Kühlmanschette 10 entlang der Linie 2d-2d in der Figur 2a.

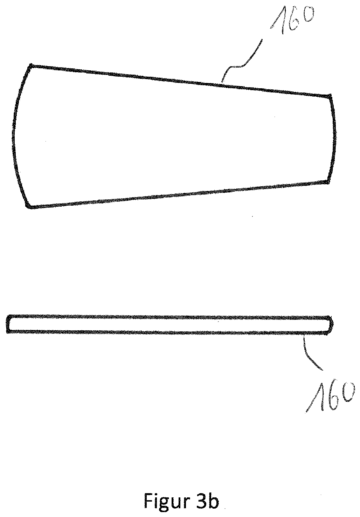

Aus der Figur 2d sind die im Sohlebereich 60 angeordnete Tasche 140 samt dem darin bereitgestellten Kühlelement 160 sowie die in den beiden Seitenbereichen des Rist-und-Spann-Bereichs 70 angeordneten Taschen 140 bzw. 141 samt den darin bereitgestellten Kühlelementen 170, 175 ersichtlich. Gemäß der Figur 2d sind die Taschen 140, 141, 142 im Lagenverbund 30 jeweils zwischen der ersten Lage 40 und der zweiten Lage 50 davon angeordnet. Es ist ferner aus der Figur 2d ersichtlich, dass die drei Taschen 140, 141, 142 einen gemeinsamen Innenraum bilden, der sich, bei angezogener Fuß-Kühlmanschette 10 entlang der Sohle und entlang der beiden Seiten des Fußes 20 erstreckt Mit weiterem Bezug auf Figur 3b kann das erste Kühlelement 160 so geformt sein, dass es, was die Länge und Breite davon betrifft, in etwa den Abmessungen einer (menschlichen) Fußsohle entspricht. Das erste Kühlelement 160 kann so in die Tasche 80 eingebracht sein bzw. werden, dass es sich parallel zur Fußsohle erstreckt und die Fußsohle von unten kühlen kann.

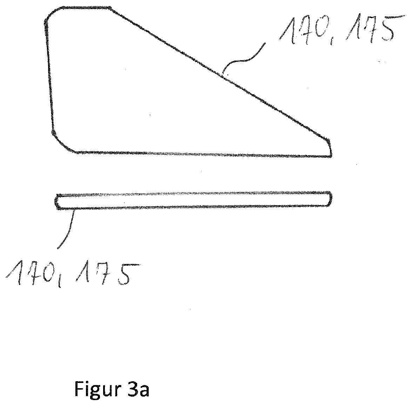

Mit Bezug auf die Figur 3a können das zweite 170 und/oder das dritte Kühlelement 175 so geformt sein, dass sie im Wesentlichen einer Seitenfläche des Fußes entsprechen, wobei sich die Kühlelemente 170, 175, was die Höhe betrifft, bis zu einem Bereich des Knöchels erstrecken können oder sich nur unter dem Knöchel erstrecken können, ohne an diesen anzustoßen. Die Kühlelemente 170, 175 können sich auch vollständig über den Knöchel erstrecken.

Gemäß der Erfindung können auch nur eine oder nur zwei Kühlelemente (d.h. z.B. nur das erste 160; oder das erste 160 und das zweite 170 oder das dritte 175; oder nur das zweite 170 und das dritte 175) in eine jeweilige Tasche 140, 141, 142 eingebracht sein, so dass eine selektive Kühlung von vorbestimmten Bereichen des Fußes möglich ist.

Ferner können das erste, zweite und dritte Kühlelement 160, 170, 175 integral (z.B. monolithisch integral) gebildet sein, so dass nur eine Kühleinrichtung (nicht gezeigt) in die Taschen 140, 141, 142 eingebracht wird, dieses aber dann sowohl die beiden Seiten als auch die Sohle des Fußes kühlen kann.

Jedes in einer Tasche bereitgestellte Kühlelement kann dazu dienen, die Temperatur des Fußes zu senken, indem es einen Wärmestrom von dem Fuß zu dem Kühlelement induziert, der mit einer Temperatursenkung des Fußes einhergeht. Der mittels eines Kühlelements erzeugte Kühleffekt kann gemäß der Erfindung alleine genutzt werden, oder er kann in Kombination mit dem Verdunstungskühlungseffekt, der mittels der kühlmittelspeichernden (z.B. wasserspeichernden) ersten Lage 40 erzielt wird, genutzt werden.

Die erfindungsgemäße Fuß-Kühlmanschette 10 kann eine Anti-Rutsch-Schicht (nicht gezeigt) an einer äußeren Oberfläche 80 davon (z.B. an bzw. auf der zweiten Lage 50 oder der dritten Lage 55) im Bereich der Sohle aufweisen, so dass sie beim normalen Gehen eines Benutzers der Fuß-Kühlmanschette 10 im Kontakt mit dem Boden kommt, um einen Reibwert zwischen dem Boden und der Fuß-Kühlmanschette 10 zu erhöhen. Die Anti-Rutsch-Schicht kann z.B. eine Profil-Sohle-Schicht sein und/oder eine flexible Schicht/Lage aus einem Material, das einen hohen Reibwert aufweist, z.B. Gummi, Kunststoff (z.B. Ethylenvinylacetat (EVA), z.B. EVA/VA (Vinylacetat), Leder, oder ähnliche Materialien. Die Anti-Rutschen-Schicht kann eine Flexibilität aufweisen, die derjenigen der ersten und/oder der zweiten Lage im Wesentlichen entspricht. Die Anti-Rutsch-Schicht bzw. Profil-Sohle-Schicht kann jedoch auch eine Flexibilität aufweisen, die geringer ist als diejenige der ersten 40 und/oder der zweiten Lage 50. Mithin kann es die Anti-Rutsch-Schicht bzw. Profil-Sohle-Schicht der erfindungsgemäßen Fuß-Kühlmanschette ermöglichen, auch außerhalb eines geschützten Innenbereichs, d.h. "im Freien" benutzt zu werden . Die Anti-Rutsch-Schicht bzw. Profil-Sohle-Schicht kann der Fuß-Kühlmanschette mechanische Stabilität geben und sie davor schützen, dass die äußere Oberfläche 80 davon mit Bodenschmutz oder Bodenfeuchtigkeit in Berührung kommt. Die Fuß-Kühlmanschette kann ferner eine Innensohle (nicht gezeigt) zum Verbessern des Komforts aufweisen. Die Innensohle kann eine dreidimensionale Negativ-Form aufweisen, die der Sohle eines menschlichen Fußes entspricht. Die Innensohle kann so angeordnet sein, dass sie sich im Wesentlichen parallel zur Fußsohle eines Benutzers erstreckt, so dass die Negativ-Form davon im Wesentlichen zur Positiv-Form der Fußsohle korrespondiert, wenn die Fuß-Kühlmanschette 10 angelegt ist bzw. benutzt wird.

Die Innensohle kann z.B. unter der ersten Lage 40 und über der zweiten Lage 50 (d.h. zwischen diesen) angeordnet sein. Die Innensohle kann z.B. auch unter der ersten und der zweiten Lage 40, 50 angeordnet sein.

Die Innensohle kann fest mit der Fuß-Kühlmanschette 10 verbunden sein, oder sie kann entfernbar bzw. austauschbar sein (Einlege-Innensohle). Die Innensohle kann in die Tasche 140 eingebracht sein, und kann sich, sofern eine Kühleinrichtung 160 bereitgestellt sind, zwischen diesen und der Fußsohle befinden.

Die Innensohle kann aus demselben Material, insbesondere Frottee, wie die erste Lage 40 bestehen oder ein solches Material aufweisen

Gemäß einer Ausführungsform kann ein Kühlelement, z.B. das Kühlelement 160, mit der Negativ-Form des Fußes bereitgestellt sein und sowohl zum Kühlen dienen als auch gleichzeitig als Innensohle dienen.

Um die erfindungsgemäße Fuß-Kühlmanschette 10 zu benutzen, kann ein Benutzter die erste Lage 40 davon in den Kontakt mit flüssigem Kühlmittel (z.B. Wasser (z.B. Wasser, bei dem es sich nicht um eine Körperflüssigkeit wie z.B. Schweiß handelt) bringen, so dass die erste Lage das Kühlmittel aufnehmen bzw. speichern kann (Schritt 100).

Zur Steigerung des Kühleffekts kann die erste Lage 40 auch mit einem anderen Kühlmittel als Wasser, z.B. mit einem Wasser-Alkohol-Gemisch oder mit einem Alkohol in Kontakt gebracht werden.

Dann kann der Benutzer über die Einstiegsöffnung 120 einen Fuß 20 mit der Fuß-Kühlmanschette 10 umfassen in dem er den Fuß in die Fußkavität 125 einbringt, so dass die erste Lage 40 in Kontakt mit dem Fuß kommt (Schritt 200). Gemäß Ausführungsformen kann es vorteilhaft sein, wenn die Haut des Fußes direkt mit der ersten Lage 40 in Kontakt kommt (d.h. ohne eine dazwischenliegende Socke oder ähnliches).

Der Benutzer kann dann die Fuß-Kühlmanschette 10 mittels der Einstiegsöffnung-Verschlusseinrichtung 130 am Fuß fixieren, so dass der Fuß im Wesentlichen fest in der Fußkavität 125 aufgenommen ist (Schritt 300).

Wenn die Fuß-Kühlmanschette 10 eine oder mehrere Taschen 140, 141, 142 aufweist, kann die Benutzung der Fuß-Kühlmanschette 10 ebenfalls das Bereitstellen von einem oder mehreren Kühlelementen 160, 170, 175 aufweisen (Schritt 400) und das Einbringen der bereitgestellten Kühlelemente 100, 110, 120 über eine Taschen-Öffnung 150 in eine jeweilige Tasche 140, 141, 142 (Schritt 500).

Wenn die Fuß-Kühlmanschette 10 eine Tasche-Verschlusseinrichtung 155 aufweist, kann die Benutzung der Fuß-Kühlmanschette 10 auch das Verschließen der Tasche-Öffnung 150 mittels der Tasche-Verschlusseinrichtung 155 aufweisen, nachdem die Kühlelemente 160, 170, 170 in die jeweilige Tasche eingebracht wurden (Schritt 600).

Die Schritte 400, 500 und 600 können vor oder nach dem Schritt 200 ausgeführt werden und auch die Reihenfolge aller hierin genannten Schritte muss nicht gemäß der hier gewählten Nummerierung erfolgen. Ebenso ist der Schritt 100 optional, z.B. dann, wenn ein Benutzer seinen Fuß nicht der Feuchtigkeit aussetzen will, sondern nur eine Kühlung mittels eines oder mehrerer Kühlelemente wünscht.

Erfindungsgemäß kann die hier beschriebene Fuß-Kühlmanschette, insbesondere wenn sie ohne Profil-Sohle-Schicht bereitgestellt ist, innerhalb eines gewöhnlichen Schuhs getragen werden, sofern der gewöhnliche Schuh so eingerichtet ist, dass gasförmiges Kühlmittel vom Inneren des gewöhnlichen Schuhs zur Umgebung gelangen kann. Um den vorhandenen Raum in einem gewöhnlichen Schuh gut zu nutzen, kann es vorteilhaft sein, eine Fuß-Kühlmanschette ohne Tasche oder zumindest ohne Kühlelement bereitzustellen, wenn sie ähnlich einer gewöhnlichen Socke in einem gewöhnlichem Schuh getragen werden soll.

Gemäß einer weiteren Ausführungsform der Erfindung kann der Lagenverbund 30 so geformt sein, dass er eine längliche Röhrenform (z.B. im Wesentlichen Hohlzylinderform) bildet, deren axiale Enden (z.B. Stirnenden) offen sind. Die Fuß-Aufnahmekavität 125 kann dann innerhalb der länglichen Röhrenform gebildet sein. Dementsprechend kann eines der offenen axialen Enden die Einstiegsöffnung 120 bilden und die Zehen eines Fußes können beispielsweise mittels des der Eintstiegsöffnung 120 gegenüberliegenden axialen Endes freiliegen. Alternativ kann dieses gegenüberliegende axiale Ende aber auch verschlossen sein, z.B. mittels entsprechendem Vernähens oder Verklebens des Lagenverbundes 30.

Die Längsachse/longitudinale Achse der Röhrenform kann einen Winkel definieren. Der Winkel kann dabei dem Winkel entsprechen, der dem Winkel zwischen der Längsachse eines

Fußes und der Längsachse eines Unterschenkels eines stehenden Benutzers oder eines liegenden Benutzers entspricht.

Die hierin beschriebene Fuß-Kühlmanschette kann eine effektive Kühlung eines Fußes mittels Verdunstungskälte und/oder mittels Kühlelementen ermöglichen, ohne dass die Kühlung einer gewünschten Körperstelle durch ein Verrutschen beeinträchtigt wird und ohne dass die Umgebung einer Feuchtigkeit/Nässe ausgesetzt wird.

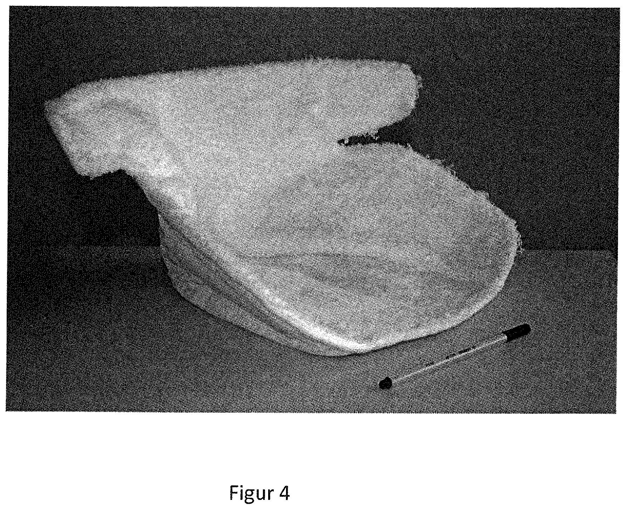

Die Figur 4 zeigt eine perspektivische Darstellung einer erfindungsgemäßen Fuß-Kühlmanschette 10 sowie einen Zeichenstift als Längenskala. Die "Baggerschaufelstruktur" der dreidimensionalen Strukur, die von dem Sohlenbereich, dem Riss-und-Spann-Bereich sowie dem Fersenbereich durch das Vorformen des Lagenverbunds, zumindest in bestimmten Abschnitten davon, erzielt werden kann, ist aus der Figur 4 ersichtlich.

## Patentansprüche

1. Fuß-Kühlmanschette (10) zum Kühlen eines Fußes,
gebildet aus einem Lagenverbund (30) mit zumindest einer ersten, textilen, saugfähigen, inneren, flexiblen Lage (40) und einer mit dieser zumindest abschnittsweise fest verbundenen zweiten, äußeren, flexiblen Lage (50),
wobei der Lagenverbund (30) unter Ausbildung einer dreidimensionalen Struktur zumindest in bestimmten Abschnitten in beständiger Weise vorgeformt ist, wobei die erste Lage (40) ein saugfähiges Textilmaterial aufweist, so dass die erste Lage (40) vor dem Anlegen der Fuß-Kühlmanschette mit flüssigem Kühlmittel zum Zweck des Kühlens des Fußes getränkt werden kann, um das Kühlmittel darin zwischen zu speichern und für eine Verdunstung durch eine Wärmeabfuhr von dem Fuß an letzterem bereitzustellen, und
wobei die zweite Lage (50) einerseits für das flüssige Kühlmittel im Wesentlichen undurchlässig ist, so dass das in der ersten Lage (40) zwischengespeicherte flüssige Kühlmittel daran gehindert wird, über eine Außenseite (80) der Fuß-Kühlmanschette (10) in flüssiger Form zu entweichen, und andererseits für das in Folge der Wärmezufuhr von dem Fuß verdampfte Kühlmittel durchlässig ist, **dadurch gekennzeichnet, dass**
die dreidimensionale Struktur eine Fußaufnahmekavität (125) definiert, die begrenzt ist von einem zentralen, länglichen, im Wesentlichen horizontalen Sohlenbereich (60) der Fuß-Kühlmanschette (10), welcher ausgebildet ist, um die Fußsohle eines Nutzers abzudecken, sowie einem Rist-und-Spann-Bereich (70) der Fuß-Kühlmanschette, welcher sich von den beiden Seitenrändern des Sohlenbereichs (60) aus nach oben erstreckt und ausgebildet ist, um den Spann und den Innen- und Außenrist des Nutzers abzudecken, und einem Fersenbereich (110) der Fuß-Kühlmanschette (10), welcher sich von einem hinteren Rand des Sohlenbereichs (60) aus nach oben erstreckt und ausgebildet ist, um die Ferse des Nutzers abzudecken, und wobei die erste Lage (40) die innerste, d.h. die im Gebrauch direkt am Fuß anliegende, Lage des Lagenverbunds (30) und der Fuß-Kühlmanschette (10) ist.

2. Fuß-Kühlmanschette gemäß Anspruch 1, wobei die erste Lage (40) aus einem Material hergestellt ist oder besteht, das aus der Gruppe ausgewählt ist, die besteht aus Frottee, Frotteefutter, Tuch, Plüsch, Samt, Velours, Vlies, Baumwolle, Viskose, insbesondere Bambus-Zellstoff-Viskose, Bambusfasern, Wolle, Wollfutter, Weblammfutter, Polmaterial, Polstermaterial, insbesondere Weichpolstermaterial, thermoplastischer Kunststoff, und/oder wobei die zweite Lage (50) aus einer wasserundurchlässigen und wasserdampfdurchlässigen Funktionstextilie hergestellt ist.

3. Fuß-Kühlmanschette (10) gemäß einem der vorangehenden Ansprüche, wobei die gesamte erste Lage (40) und/oder die gesamte zweite Lage (50) durchgängig und insbesondere monolithisch ausgebildet sind, so dass sie sich durchgängig bzw. monolithisch über den Sohlenbereich (60), Rist-und-Spann-Bereich (70) und Fersen-Bereich (110) hinweg erstrecken.

4. Fuß-Kühlmanschette (10) gemäß einem der vorangehenden Ansprüche, wobei
die Fuß-Kühlmanschette (10) bzw. deren dreidimensionale Struktur in Längsrichtung vorne offen bzw. kappenfrei ausgebildet ist, so dass der vordere Rand bzw. die Stirnseite des Sohlenbereichs (60) freiliegt bzw. die Fußaufnahmekavität (125) von vorne/stirnseitig frei zugänglich ist, und/oder
die Fuß-Kühlmanschette (10) bzw. deren dreidimensionale Struktur oben mittig offen bzw. zungenfrei ausgebildet ist, so dass die Fußaufnahmekavität (125) unter Ausbildung einer über dem Sohlenbereich (60) angeordneten Einstiegsöffnung (120), welche sich insbesondere entlang der gesamten Längsachse des Sohlenbereichs (60) erstreckt, von oben frei zugänglich ist bzw, der Rist-und-Spann-Bereich (70) in einen ersten/linken (90) und einen zweiten/rechten Flügelabschnitt (100) unterteilt ist, die sich von einem jeweiligen Seitenrand des Sohlenbereichs (60) aus nach oben erstrecken, wobei die beiden Flügelabschnitte (90, 100) insbesondere über den Fersenbereich (110) miteinander verbunden sind, und/oder
wobei die Fuß-Kühlmanschette (10) bzw. deren dreidimensionale Struktur im Wesentlichen eine Baggerschaufelstruktur hat.

5. Fuß-Kühlmanschette gemäß einem der vorangehenden Ansprüche, wobei ein Bereich zwischen dem jeweiligen Seitenrand des Sohlenbereichs (60) und dem Rist-und-Spann-Bereich (70) in beständiger Weise vorgeformt ist.

6. Fuß-Kühlmanschette gemäß einem der vorangehenden Ansprüche, wobei der Lagenverbund (30) eine einzelne Tasche oder eine Mehrzahl an Taschen (140, 141, 142) aufweist, wobei jede Tasche (140, 141, 142) eingerichtet ist, um jeweils ein oder mehrere Kühlelemente (160, 170, 175) aufzunehmen.

7. Fuß-Kühlmanschette (10) gemäß Anspruch 6, wobei
zumindest eine Tasche (140) in dem Sohlenbereich (60) der Fuß-Kühlmanschette (10) angeordnet ist und sich entlang eines Längsabschnitts davon erstreckt und/oder
zumindest eine Tasche (141) in einem medialen Seitenabschnitt des Rist-und-Spann-Bereichs (70) angeordnet ist und sich entlang eines Längsabschnitts davon erstreckt und/oder
zumindest eine Tasche (142) in einem lateralen Seitenabschnitt des Rist-und-Spann-Bereichs (70) angeordnet ist und sich entlang eines Längsabschnitts davon erstreckt,

8. Fuß-Kühlmanschette (10) gemäß einem der vorangehenden Ansprüche, wobei der Lagenverbund (30) zusätzlich eine dritte, flexible Lage (55) aufweist, die mit der zweiten Lage (50) zumindest abschnittsweise fest verbunden ist und die die Außenfläche (80) der Fuß-Kühlmanschette (10) im Wesentlichen vollständig bildet, und wobei die dritte Lage (55) durchlässig für gasförmiges Kühlmittel und z.B. auch durchlässig für flüssiges Kühlmittel ist.

## Claims

1. Foot cooling cuff (10) for cooling a foot,
formed by a layer compound (30) having at least a first, textile, absorbent, inner, flexible layer (40) and a second, outer, flexible layer (50) firmly connected thereto at least in sections,
wherein the layer compound (30) is pre-formed in an enduring way at least in particular sections, thereby forming a three-dimensional structure,
wherein the first layer (40) comprises an absorbent textile material, so that the first layer (40) may be soaked with liquid coolant before the foot cooling cuff is applied for the purpose of cooling the foot, for an intermediate storing of the coolant therein and for providing it for an evaporation by heat dissipation from the foot to the latter, and
wherein the second layer (50) is, on the one hand, substantially impermeable to the liquid coolant, so that the liquid coolant intermediately stored in the first layer (40) is prevented from escaping via an outer side (80) of the foot cooling cuff (10) in liquid form, and, on the other hand, permeable to the coolant evaporated as a result of the heat supply from the foot, **characterized in that**
the three-dimensional structure defines a foot receiving cavity (125) which is limited by a central, elongate, substantially horizontal sole region (60) of the foot cooling cuff (10), which is configured to cover the sole of the foot of a user, as well as an instep region (70) of the foot cooling cuff which extends upwards from both side edges of the sole region (60) and is configured to cover the instep and the inner instep and the outer instep of the user, and a heel region (110) of the foot cooling cuff (10) extending upwards from the rear edge of the sole region (60) and configured to cover the heel of the user, and
wherein the first layer (40) is the innermost layer of the layer compound (30) and of the foot cooling cuff, i.e. the layer directly resting against the foot.

2. Foot cooling cuff according to claim 1, wherein the first layer (40) is made of or consists of a material which is selected from the group consisting of terry cloth, terry cloth lining, cloth, plush, velvet, velour, non-woven material, cotton, viscose, in particular bamboo cellulose viscose, bamboo fibers, wool, wool lining, woven lamb lining, pile material, padding material, in particular soft padding material, thermoplastic resin, and/or wherein the second layer (50) is made of a water-impermeable and water vapor permeable functional textile.

3. Foot cooling cuff (10) according to any one of the preceding claims, wherein the entire first layer (40) and/or the entire second layer (50) are formed to be continuous and in particular monolithic, so that they extend continuously and/or monolithically over the sole region (60), the instep region (70) and the heel region (110).

4. Foot cooling cuff (10) according to any one of the preceding claims, wherein
the foot cooling cuff (10) and its three-dimensional structure, respectively, is formed to be open at the front and cap-free, respectively, so that the front edge and the front face, respectively, of the sole region (60) is exposed and the foot receiving cavity (125) is freely accessible from the front side/front face, respectively, and/or
the foot cooling cuff (10) and its three-dimensional structure, respectively, is formed to be open at the center top and tongue-free, respectively, so that the foot receiving cavity (125) is freely accessible from the top, thereby forming an access opening (120) disposed above the sole region (60), which extends in particular along the entire longitudinal axis of the sole region (60), and the instep region (70) is divided into a first/left-hand (90) and a second/right-hand wing portion (100), respectively, which extend in an upward direction from a respective side edge of the sole region (60), wherein the two wing portions (90, 100) are connected to each other especially through the heel region (110), and/or
wherein the foot cooling cuff (10) and its three-dimensional structure, respectively, substantially has an excavator bucket structure.

5. Foot cooling cuff according to any one of the preceding claims, wherein a region between the respective side edge of the sole region (60) and the instep region (70) is pre-formed in an enduring way.

6. Foot cooling cuff according to any one of the preceding claims, wherein the layer compound (30) comprises a single pocket or a plurality of pockets (140, 141, 142), wherein each pocket (140, 141, 142) is configured to receive respectively one or more cooling elements (160, 170, 175).

7. Foot cooling cuff (10) according to claim 6, wherein
at least one pocket (140) is arranged in the sole region (60) of the foot cooling cuff (10) and extends along a longitudinal portion thereof, and/or
at least one pocket (141) is arranged in a medial side portion of the instep region (70) and extends along a longitudinal portion thereof, and/or
at least one pocket (142) is arranged in a lateral side portion of the instep region (70) and extends along a longitudinal portion thereof.

8. Foot cooling cuff (10) according to any one of the preceding claims, wherein the layer compound (30) additionally comprises a third, flexible layer (55) which is firmly connected to the second layer (50) at least in sections and which substantially entirely constitutes the outer surface (80) of the foot cooling cuff (10), and wherein the third layer (55) is permeable to gaseous coolant and, for example, is also permeable to liquid coolant.

## Revendications

1. Manchette de refroidissement de pied (10) pour refroidir un pied,
formée d'un assemblage de couches (30) présentant au moins une première couche (40) flexible, intérieure, absorbante, textile, et une deuxième couche (50) flexible, extérieure, reliée à celle-ci au moins en sections,
dans laquelle l'assemblage de couches (30) est préformé au moins en certaines portions de manière persistante en formant une structure tridimensionnelle,
dans laquelle la première couche (40) comprend un matériau textile absorbant, de sorte que la première couche (40) peut être imprégnée d'un réfrigérant liquide pour refroidir le pied avant l'application de la manchette de refroidissement de pied, pour le stockage temporaire du réfrigérant là-dedans et pour la mise à disposition pour une évaporation par dissipation thermique depuis le pied à ce dernier, et
dans laquelle la deuxième couche (50) est d'une part essentiellement imperméable au réfrigérant liquide, de sorte que le réfrigérant liquide stocké temporairement dans la première couche (40) est empêché d'échapper en forme liquide à travers une surface extérieure (80) de la manchette de refroidissement de pied (10), et d'autre part perméable au réfrigérant évaporé depuis le pied en raison de l'apport de chaleur, **caractérisée en ce que**
la structure tridimensionnelle définit une cavité de réception de pied (125) qui est limitée par une région de semelle (60) centrale, allongée et essentiellement horizontale de la manchette de refroidissement de pied (10), qui est configurée pour recouvrir la plante du pied d'un utilisateur, ainsi que par une région de cou-de-pied (70) de la manchette de refroidissement de pied, qui s'étend à partir des deux bords latéraux de la région de semelle (60) vers le haut et est configurée pour recouvrir le cou-de-pied ainsi que le cou-de-pied intérieur et extérieur de l'utilisateur, et par une région de talon (110) de la manchette de refroidissement de pied (10) qui s'étend depuis un bord arrière de la région de semelle (60) vers le haut et est configurée pour recouvrir le talon de l'utilisateur, et
dans laquelle la première couche (40) est la couche de l'assemblage de couches (30) et de la manchette de refroidissement de pied (10) la plus intérieure, à savoir la couche directement adjacente au pied.

2. Manchette de refroidissement de pied selon la revendication 1, dans laquelle la première couche (40) est fabriquée ou faite d'un matériau choisi dans le groupe constitué par le tissu éponge, la doublure en tissu éponge, le tissu, la peluche, le velours, le non-tissé, le coton, la viscose, en particulier la viscose de cellulose de bambou, les fibres de bambou, la laine, la doublure en laine, la doublure en tissu de peau d'agneau, le matériau de poil, la matière de rembourrage, en particulier la matière de rembourrage molle, la résine thermoplastique, et/ou dans laquelle la deuxième couche (50) est fabriquée en un textile fonctionnel imperméable à l'eau et perméable à la vapeur d'eau.

3. Manchette de refroidissement de pied (10) selon l'une quelconque des revendications précédentes, dans laquelle la première couche entière (40) et/ou la deuxième couche entière (50) sont configurées de manière cohérente et en particulier monolithique, de manière à s'étendre de manière cohérente, respectivement monolithique, par-dessus de la région de semelle (60), la région de cou-de-pied (70) et la région de talon (110).

4. Manchette de refroidissement de pied (10) selon l'une quelconque des revendications précédentes, dans laquelle
la manchette de refroidissement de pied (10), respectivement sa structure tridimensionnelle, est configurée de manière à être ouverte vers l'avant, respectivement sans capuchon, de sorte que le bord avant, respectivement la face frontale, de la région de semelle (60) est exposé, respectivement la cavité de réception de pied (125) est librement accessible par le devant / la face frontale, et/ou
la manchette de refroidissement de pied (10), respectivement sa structure tridimensionnelle, est configurée de manière à être ouverte au milieu en haut, respectivement sans languette, de sorte que la cavité de réception de pied (125) est librement accessible par le haut, en formant une ouverture d'entrée (120) disposée au-dessus de la région de semelle (60) qui s'étend particulièrement le long de l'axe longitudinal entier de la région de semelle (60), respectivement la région de cou-de-pied (70) est divisée en une première partie d'ailette / partie d'ailette gauche (90) et une deuxième partie d'ailette / partie d'ailette droite (100) qui s'étendent à partir d'un bord latéral respectif de la région de semelle (60) vers le haut, les deux parties d'ailette (90, 100) étant en particulier reliées l'une à l'autre par la région de talon (110), et/ou
dans laquelle la machette de refroidissement de pied (10), respectivement sa structure tridimensionnelle, présente essentiellement une structure de godet d'excavation.

5. Manchette de refroidissement de pied selon l'une quelconque des revendications précédentes, dans laquelle une région entre le bord latéral respectif de la région de semelle (60) et la région de cou-de-pied (70) est préformée de manière persistante.

6. Manchette de refroidissement de pied selon l'une quelconque des revendications précédentes, dans laquelle l'assemblage de couches (30) comprend une seule poche ou une pluralité de poches (140, 141, 142), dans laquelle chaque poche (140, 141, 142) est configurée pour recevoir respectivement un ou plusieurs éléments de refroidissement (160, 170, 175).

7. Manchette de refroidissement de pied (10) selon la revendication 6, dans laquelle
au moins une poche (140) est disposée dans la région de semelle (60) de la manchette de refroidissement de pied (10) et s'étend le long d'une partie longitudinale de celle-ci, et/ou
au moins une poche (141) est disposée dans une partie de côté médiale de la région de cou-de-pied (70) et s'étend le long d'une partie longitudinale de celle-ci, et/ou
au moins une poche (142) est disposée dans une partie de côté latérale de la région de cou-de-pied (70) et s'étend le long d'une partie longitudinale de celle-ci.

8. Manchette de refroidissement de pied (10) selon l'une quelconque des revendications précédentes, dans laquelle l'assemblage de couches (30) comprend en outre une troisième couche flexible (55) qui est reliée de manière fixe à la deuxième couche (50) au moins en sections et qui constitue la surface extérieure (80) de la manchette de refroidissement de pied (10) essentiellement entièrement, et dans laquelle la troisième couche (55) est perméable à un réfrigérant sous forme gazeuse et est, par exemple, également perméable à un réfrigérant liquide.
